# NEUE EUROPÄISCHE PATENTSCHRIFT

(11) **EP 1 532 975 B2**
(45) Veröffentlichungstag und Bekanntmachung des Hinweises auf die Entscheidung über den Einspruch: **02.12.2009**
(45) Hinweis auf die Patenterteilung: 28.02.2007
(21) Anmeldenummer: 03026546.6
(22) Anmeldetag: 18.11.2003
(51) Int. Cl.: A61K 9/18, A61K 31/40

(54) **Verfahren zur Herstellung von freifliessenden, pulverförmigen Atorvastatin-Adsorbaten**
Process for the manufacture of free-flowing, powdery atorvastatin adsorbates
Procédé pour la préparation d' adsorbats d'atorvastatine pulverulents à écoulement libre

(43) Veröffentlichungstag der Anmeldung: 25.05.2005
(60) Teilanmeldung: 05013829.6
(73) Patentinhaber: Helm AG, 20097 Hamburg (DE)
(72) Erfinder: Doser, Karl-Heinz, Dr, 21244 Buchholz i.d. Nordheide (DE); Glänzer, Klaus, Dr., 22337 Hamburg (DE); Waldraff, Robert, Dr., 87700 Memmingen (DE)
(74) Vertreter: Straus, Alexander

(56) Entgegenhaltungen:
- WO-A-03/000238
- WO-A1-2004//110407
- US-B1- 6 248 363
- DOELKER E: "MODIFICATIONS CRISTALLINES ET TRANSFORMATIONS POLYMORPHES AU COURS DES OPERATIONS GALENIQUES CRYSTALLINE MODIFICATIONS AND POLYMORPHISM CHANGES DURING DRUG MANUFACTURE" ANNALES PHARMACEUTIQUES FRANCAISES, MASSON, PARIS, FR, Bd. 60, Nr. 3, 2002, Seiten 161-176, XP009003219 ISSN: 0003-4509
- KATZHENDLER I ET AL: "Crystalline properties of carbamazepine in sustained release hydrophilic matrix tablets based on hydroxypropyl methylcellulose" JOURNAL OF CONTROLLED RELEASE, ELSEVIER SCIENCE PUBLISHERS B.V. AMSTERDAM, NL, Bd. 54, Nr. 1, 1. Juni 1998 (1998-06-01), Seiten 69-85, XP004127851 ISSN: 0168-3659
- FALBE, JÜRGEN,PROF. DR. & REGITZ, MANFRED, PROF., DR.: 'Römpp Chemie Lexikon', 1990, GEORG THIEME VERLAG, STUTTGART, ISBN 3-13-734809-9 Seite 2430
- W. A. RITSCHEL ET AL: 'Die Tablette, 2. Auflage', 2002, EDITIO CANTOR VERLAG, AULENDORF, ISBN 3-87193-228-0 Seiten 354 - 355

## Beschreibung

Die vorliegende Erfindung betrifft ein neuartiges Verfahren zur Herstellung von Atorvastatin-Adsorbaten und von deren Hydraten bzw. Solvaten. Die erfindungsgemäßen Atorvastatin-Adsorbate enthalten in einer besonders bevorzugten Ausführungsform den Wirkstoff in fein verteilter amorpher Form. Besonders bevorzugt liegt der Wirkstoff als Erdalkalisalz, insbesondere als Hemicalcium-Salz sowie als dessen Hydrat-Formen, vor.

Die Erfindung betrifft weiterhin Atorvastatin-Adsorbate und dessen Hydrate bzw. Solvate, die gemäß dem vorgenannten Verfahren herstellbar sind.

Die Erfindung betrifft schließlich auch pharmazeutische Formulierungen, zu deren Herstellung die vorgenannten Atorvastatin-Adsorbate eingesetzt werden. Bevorzugte erfindungsgemäße Arzneiformen sind Tabletten, Kapseln, Pellets und Granulate, die auf an sich bekannte Weise mit üblichen, pharmazeutisch akzeptablen Hilfsstoffen hergestellt werden. Erfindungsgemäß besonders bevorzugt sind den Wirkstoff schnell freisetzende Tabletten, die durch Direktverpressung der erfindungsgemäßen Atorvastatin-Adsorbate hergestellt werden.

Der unter dem INN Atorvastatin bekannte Wirkstoff ist dem Chemiker auch als Calcium-Salz der [R-(R*,R*)]-2-(4-Fluorphenyl)-β,δ-dihydroxy-5-(1-methyl-ethyl)-3-phenyl-phenylamino)-carbonyl]-1H-pyrrol-1-heptansäure geläufig. Dieser Wirkstoff ist ein ausgezeichneter Hemmer des Enzyms 3-Hydroxy-3-methylglutaryl-(Coenzym A)-Reduktase I, auch bekannt unter dem Akronym HMG-CoA-Reduktase; er ist somit als hypolipidämischer und hypocholesterinämischer Wirkstoff zur Therapie von Lipidstoffwechselstörungen verwendbar.

HMG-CoA-Reduktase-Hemmer werden daher auch erfolgreich zur Vorbeugung und zur Behandlung von koronarer Herzerkrankung und anderen Erkrankungen, die auf arteriosklerotischen Gefässveränderungen beruhen, eingesetzt. Über verschiedene Wirkmechanismen kann dabei eine signifikante Lipidsenkung, speziell des Cholesterins, im Plasma erzielt werden (siehe hierzu z.B. Milestones in Drug Therapy / HMG-CoA-Reductase Inhibitors, Hrsg. G. Schmitz und M. Torzewski, Birkhäuser Verlag Basel-Boston-Berlin [2002]).

Die chemische Struktur des Atorvastatins wurde in ihrer racemischen Form erstmals in der EP 247 633 beschrieben. Das Hemicalcium-Salz der als Wirkstoff verwendeten aktiven [R-(R*,R*)]-Form wurde erstmalig in der EP 409 281 offengelegt und als Feststoff beschrieben; diese Schrift enthält aber keine Offenbarung betreffend einer eventuellen Kristallinität des Produktes.

Die Herstellung des Atorvastatins und von wichtigen Zwischenprodukten, wie z. B. der Lacton-Vorstufe des Atorvastatins, wird in verschiedenen Patentschriften erwähnt, so z. B. in EP-A-330 172, EP-A-553 213, EP-A-687 263, EP-A-915 866, EP-A-1 054 860, WO 99/57109 A, WO 01/72706 A, WO 02/55519 A.

Aus dem Stand der Technik ist bekannt, dass Atorvastatin in Form seines Hemicalcium-Salzes nicht nur als amorpher Feststoff, wie bereits in der EP-A-409 281 beschrieben, vorliegt, sondern auch in mehr als 30 kristallinen polymorphen Formen anfallen kann.

So beschreiben die WO 97/03958 A sowie die zeitgleich eingereichte WO 97/03959 A die kristallinen Formen III sowie I, II und IV des Hemicalcium-Salzes des Atorvastatins. Während die Form III durch Inkubation der Form II für 11 Tage in einer Atmosphäre mit einem Feuchtigkeitsgehalt von 95 % erhalten wird, können die Formen I, II und IV alle aus Methanol bzw. aus Methanol-Wasser-Gemischen in verschiedenen Mischungsverhältnissen und bei unterschiedlichen Temperaturen erhalten werden. Sehr oft kann die gewünschte polymorphe Form gemäß diesen Dokumenten auch nur durch einen massiven Einsatz von Impfkristallen erhalten werden.

Die WO 01/36384 A legt die polymorphe Form V des Atorvastatins in Form seines Hemicalcium-Salzes offen, die ebenfalls aus einem Methanol-Wasser-Gemisch erhalten wird.

Die WO 02/57229 A beschreibt ebenfalls eine polymorphe Form V des Atovastatins, herstellbar durch Rühren einer Suspension aus rohem Atorvastatin-Hemicalcium in einem Gemisch aus Wasser und absolutem Ethanol im Verhältnis 14:3. Nach den in den Schriften offengelegten Charakterisierungen sind diese beiden Formen aber unterschiedlich.

Die WO 02/41834 A beschreibt die polymorphe Form VII des Atorvastatins in Form seines Hemicalcium-Salzes, die durch Rühren einer Suspension der polymorphen Form V oder der polymorphen Form I des Atorvastatins in Form seines Hemicalcium-Salzes in absolutem Ethanol bei Raumtemperatur erhalten werden kann.

Die WO 02/43732 A legt die polymorphen Formen VI, VIII, IX, X, XI und XII des Atorvastatins in Form seines Hemicalcium-Salzes offen, wobei die Form VI aus wäßrigem Aceton, die Form VIII aus Ethanol oder aus n-Butanol, die Form IX aus Ethanol oder aus n-Butanol, die Form X aus wäßrigem Ethanol, die Form XI aus Methylethylketon und die Form XII aus wäßrigem Ethanol erhalten werden können. Im Falle gleicher Lösungsmittel ist festzustellen, dass der Erhalt einer bestimmten polymorphen Form von geringfügig geänderten Bedingungen, wie unterschiedlichen Temperaturen oder einem speziellen Wassergehalt abhängig ist.

Die WO 02/51804 A benutzt eine zur Beschreibung der polymorphen Formen des Atorvastatins zu den obigen Patentdokumenten unterschiedliche Terminologie. Sie beschreibt die polymorphen Formen X, A, B1, B2, C, D und E des Atorvastatins in Form seines Hemicalcium-Salzes, wobei die Form X aus Methanol und Methyl-t-butylether, die Form A aus Isopropanol, welches Spuren von Wasser enthält, die Form B1 aus einem Acetonitril-THF-Gemisch, die Form B2 aus reinem Acetonitril, die Form C aus wäßrigem Isopropanol, die Form D aus Ethanol und die Form E aus Methylethylketon durch Ausfällen mit Heptan erhalten werden können. Auch hier ist wieder festzustellen, dass bei gleichen Lösungsmitteln geringfügige Änderungen bei den sonstigen Bedingungen schon zur Kristallisation einer anderen polymorphen Form führen.

Die WO 03/04470 A beschreibt schließlich die polymorphen Formen V, VI, VII, VIII, IX, X, XI, XII, XIII, XIV, XV, XVI, XVII, XVIII und XIX des Atorvastatins in Form seines Hemicalcium-Salzes, wobei die Form V, die als Trihydrat charakterisiert ist, aus wäßrigem Acetonitril, die Form VI aus wäßrigem DMF, die Form VII, die als Sesquihydrat charakterisiert wird, aus wäßrigem Aceton, die Form VIII, bei der es sich um ein Dihydrat handelt, wieder aus wäßrigem DMF, und die Form IX wieder aus wäßrigem Aceton erhalten wird. Die Form X, bei der es sich um ein Trihydrat handelt, ist aus wäßrigem Isopropanol erhältlich, die Form XI wieder aus wäßrigem Acetonitril, die Form XII aus THF-haltigem Wasser, die Form XIII aus wäßrigem Methanol und die Form XIV, bei der es sich um ein Hexahydrat handelt, wieder aus wäßrigem Isopropanol. Die Form XV, beschrieben als Trihydrat, fällt aus wäßrigem Aceton an, die Form XVI, die als Tetrahydrat-Acetonitril-Solvat beschrieben wird, aus wäßrigem Acetonitril. Die Form XVIII, bei der es sich um ein Solvat handelt, aus einem DMF-Wasser-Acetonitril-Gemisch und die Form XIX, bei der es sich ebenfalls um ein Solvat handelt, aus Methylethylketon. Die Form XVII, bei der es sich um ein Tetrahydrat handelt, schließlich wird durch längeres Trocknen der Form XVI erhalten. Auch aus dieser Offenlegung wird wieder deutlich, dass geringfügige Änderungen der Kristallisationsbedingungen zu anderen polymorphen Formen bei der Verwendung gleicher Lösungsmittelsysteme führen können. Es ist auch bei diesem Patentdokument festzustellen, wie bei den oben zitierten Offenlegungen zur Kristallisation des Atorvastatin-Hemicalcium-Salzes, dass die Entstehung von definierten polymorphen Formen auch von dem Ausgangspolymorph, eventuell auch von Impfkristallen und von den unterschiedlichen Trocknungsbedingungen stark abhängig ist.

Zusammenfassend kann daher festgestellt werden, dass die Kristallisation von Atorvastatin-Hemicalcium in einer definierten polymorphen Form außerordentlich stark von geringfügig geänderten Prozessparametern abhängig ist, was zu einer sehr aufwendigen Prozesssteuerung führt, da ein solches definiertes Polymorph für einen pharmazeutischen Wirkstoff unbedingt sicherzustellen ist, um den regulatorischen Anforderungen an Arzneimittel gerecht zu werden und natürlich auch um die gleichbleibende Qualität des Arzneimittels und damit die Einnahmesicherheit für den Patienten zu gewährleisten.

Eine Möglichkeit, dieses Problem zu lösen und zu einem vorteilhafteren Isolationsprozess zu kommen, ist die Verwendung von amorphem Atorvastatin-Hemicalcium. Zur Gewinnung der amorphen Form sind im Stand der Technik mehrere Verfahren beschrieben, so z. B. in der EP-A-839 132: aus einer Lösung des Wirkstoffes in einem THF-Toluol-Gemisch durch Entfernen des Lösungsmittels, in der EP-A-1 185 264: aus einer Lösung des Wirkstoffes in THF durch Ausfällen des Produktes mit Heptan oder gemäß der EP-A-1 235 800: durch "Umkristallisation" des Wirkstoffes aus niederen Alkoholen.

Die Verwendung von amorphem Atorvastatin-Hemicalcium als Wirkstoff in pharmazeutischen Formulierungen bringt zum einen das Problem der im Vergleich zu kristallinem Wirkstoff geringeren Stabilität der amorphen Form gegenüber Umfeldbedingungen, wie Sauerstoff, Licht, Wärme und Restfeuchtigkeit, sowie der höheren Empfindlichkeit in Bezug auf stabilitätsmindernde Wechselwirkungen mit pharmazeutischen Hilfs- und Zusatzstoffen mit sich. So ist z. B. bekannt, dass sich Atorvastatin-Hemicalcium in Gegenwart von Feuchtigkeit und leicht sauer reagierenden Substanzen sehr leicht in das Atorvastatin-Lacton umwandelt, was zu einer nicht akzeptablen Wirkstoffabnahme in der fertigen Tablette führen kann. Um den Einfluss derartiger Reaktionen in der fertigen Arzneiform zu verhindern, wurden meist alkalisierende Zusatzstoffe wie etwa bestimmte Alkali- und Erdalkaliverbindungen, alkalische Puffersysteme (siehe z. B. US-A-5,180,589, US-A-5,686,104, WO 00/35425 A und WO 01/93859A) oder auch die Verwendung polymerer Verbindungen mit Amido- oder Aminogruppen (Polyvinylpyrrolidone oder Cholestyramine, siehe WO 01/76566 A) zur Stabilisierung vorgeschlagen. Eine Kombination beider Stabilisierungsarten (Natriumhydroxid plus Polyvinylpyrrolidon) wurde für analoge Statin-Verbindungen ebenfalls verwendet (WO 98/57917 A).

WO 03/000238 A1 beschreibt Adsorbate von amorphen Wirkstoffen. Auf S. 13, Z. 18/19 wird in einer großen Liste von Substanzen auch Atorvastatin Calcium aufgeführt. Zur Herstellung von Adsorbaten der aufgeführten Wirkstoffe dienen Cellulosederivate oder anorganische Stoffe wie SiO₂, TiO₂, Al₂O₃. Diese Adsorbermaterialien sind allerdings zur Herstellung einer Atorvastatin-Formulierung ungeeignet. Versuche haben gezeigt, dass Adsorbate resultieren, aus denen entweder der Wirkstoff nur ungenügend freigesetzt wird oder sich bei höherer Temperatur zersetzt.

Ein weiteres Problem der Verwendung von amorphem Atorvastatin-Hemicalcium als Wirkstoff, ist die Tatsache, dass nach den experimentellen Erfahrungen der Erfinder der vorliegenden Anmeldung beim Fällungsprozess der amorphen Form öfter auch heterogene Produkte: ein Teil kristallin und ein anderer Teil amorph, erhalten werden, was dazu führt, dass der Fällungsprozess wiederholt werden muss. Jede zusätzliche Herstellungsschritt birgt aber das Risiko eines Substanzverlustes von ca. 5 % bis 10 % in sich, was aus ökonomischer Sicht für Wirkstoffe wie Atorvastatin-Hemicalcium, das in einer langwierigen, aufwendigen und teuren Synthesefolge hergestellt werden muss, sicher nicht erwünscht ist.

Aus der DE 10008506 A1 ist zwar für einen analogen Statin-Wirkstoff , nämlich Cerivastatin, ein Verfahren zur Herstellung eines Wirkstoffgranulats bekannt, das einen solchen Substanzverlust vermeidet, wobei eine ausschließlich wäßrige Wirkstofflösung in Gegenwart eines Füllstoffes und eines Bindemittels direkt granuliert wird und das so erhaltene Granulat nach dem Trocknen in Tabletten weiter verarbeitet wird. Für das im vorliegenden Fall bestehende Problem hinsichtlich des Wirkstoffs Atorvastatin-Hemicalcium ist dies aber keine technisch durchführbare Lösung, da in den in DE 10008506 A1 beschriebenen Granulaten der Wirkstoff in einer maximalen Menge von 0,5 % (w/w) vorliegt. Für therapeutische Dosierungen des Atorvastatin-Hemicalciums muss der Wirkstoffgehalt aber zwischen 20 mg und 80 mg liegen. Dies würde gemäß der Offenbarung der DE 10008506 A1 zu Tablettengewichten zwischen 4 und 16 g führen. Der akzeptable Bereich für Tabletten, die zur oralen Einnahme geeignet sind, d. h. vom Patienten geschluckt werden müssen, liegt zwischen 100 mg und 1 g.

Die bekannten Verfahren zur Herstellung von pharmazeutischen Formulierungen des Atorvastatin-Hemicalciums sind, soweit sie überhaupt durchführbar sind, technisch daher sehr aufwendig, zeit- und kostenintensiv und lösen das Problem einer stabilen Arzneiform bislang nicht oder nicht zufriedenstellend. Letzteres trifft ganz besonders auf das wegen seiner besseren Herstellbarkeit und wegen guter Auflöseeigenschaften bevorzugte Atorvastatin-Hemicalcium mit großer spezifischer Oberfläche, d. h. insbesondere amorph oder feinpulvrig, zu. In solchen Fällen reichen die bekannten, zuvor ausgeführten Stabilisierungen des Standes der Technik nicht aus.

Daher ist es die Aufgabe der vorliegenden Erfindung, ein einfaches und kostengünstiges Verfahren zur Herstellung von stabilen Atorvastatin-Pulversystemen zu entwickeln, die direkt zur Herstellung von pharmazeutischen Formulierungen eingesetzt werden können, wobei dieses Verfahren jedoch nicht auf eine besonders bevorzugte Wirkstoffmorphologie eingeschränkt ist, welches die oben diskutierten Nachteile vermeidet.

Diese Aufgabe wird erfindungsgemäß durch ein Verfahren zur Herstellung von Atorvastatin-Adsorbaten gemäß Anspruch 1 gelöst. Dabei geht man von einer Lösung des Atorvastatins oder seiner Salze in einem vorwiegend organischen Lösungsmittel aus, wobei der Wirkstoff in den organischen Lösungsmitteln gelöst vorliegt, suspendiert darin das Adsorbermaterial und entfernt das Lösungsmittel durch Trocknen. Der Gesamtwassergehalt des Lösungsmittels beträgt erfindungsgemäß nicht mehr als 10 Vol.-%, bevorzugt nicht mehr als 5 Vol.-% (vergleiche Anspruch 1).

In einer bevorzugten Ausführungsform der Erfindung enthalten die Atorvastatin-Adsorbate den Wirkstoff in fein verteilter amorpher Form, insbesondere als Calciumsalz. Das amorphe Atorvastatin gemäß der vorliegenden Erfindung kann sowohl in wasserfreier Form als auch in Form von Solvaten bzw. Hydraten vorliegen.

Die Erfindung betrifft weiterhin nach dem vorgenannten Verfahren herstellbare Atorvastatin-Adsorbate und dessen Solvate bzw. Hydrate. Die Erfindung betrifft weiterhin pharmazeutische Formulierungen enthaltend diese neuen Atorvastatin-Adsorbate. Die pharmazeutischen Formulierungen enthalten gegebenenfalls weitere Hilfsstoffe und können in die gewünschte Darreichungsform überführt werden. Besonders bevorzugt sind den Wirkstoff schnell freisetzende, durch Direktverpressung hergestellte Tabletten.

Für das erfindungsgemäße Verfahren zur Herstellung der Atorvastatin-Adsorbate eignen sich organische Lösungsmittel für die den pharmazeutischen Wirkstoff enthaltende Lösung. Die organischen Lösungsmittel sind insbesondere ausgewählt aus der Gruppe der niederen Alkanole mit 1 bis 4 Kohlenstoffatomen, der Gruppe der Ether und der Gruppe der aliphatischen Ketone und Gemische der vorgenannten Lösungsmittel. Besonders bevorzugt sind Methanol, Ethanol, Isoproponol, n-Propanol, Aceton und andere Lösungsmittel wie Ethylacetat, Methylethylketon, MTBE (Methyltert.-butylester) und Gemische aus Ethylacetat und Hexan sowie Gemische der vorgenannten Lösungsmittel.

Als Absorbermaterialien werden erfindungsgemäß pharmazeutisch akzeptable Hilfsstoffe eingesetzt, die für eine schnelle Wirkstofffreisetzung geeignet sind, wie Polyole, insbesondere Mannit, Zucker und Zuckerderivate, insbesondere Lactose, oder Gemische der vorgenannten Substanzen. Lactose und Mannit sind erfindungsgemäß bevorzugt.

Das Verhältnis des pharmazeutischen Wirkstoffes zum Adsorbermaterial liegt erfindungsgemäß im Bereich von 1:0,1 bis 10, wobei insbesondere ein Bereich von 1:1 bis 1:2 bevorzugt wird.

Für die Herstellung der pharmazeutischen Formulierungen, wobei insbesondere Tabletten bevorzugt sind, können alle üblichen pharmazeutischen Hilfsstoffe verwendet werden. Als Füllstoffe können z.B. Cellulosen und Cellulosederivate (z.B. mikrokristalline Cellulose, native Cellulose, Hydroxypropylcellulose, Hydroxypropylmethylcellulose, Methylcellulose), Zucker (z. B. Lactose, Fructose, Saccharose, Glukose, Maltose), Zuckeralkohole (z.B. Lactit Mannit, Sorbit, Xylit) anorganische Füllstoffe (z.B. Calciumphosphate und Calciumsulfate) und Stärken (z.B. Maisstärke, Kartoffelstärke, Weizenstärke, Dextrine, pregelatinisierte Stärken) verwendet werden. Darüber hinaus können alle weiteren Hilfsstoffe, die dem Fachmann aufgrund seines galenischen Basiswissens bekannt sind, wie z.B. Schmiermittel, Zerfallshilfsmittel, Netzmittel, alkalische Zusatzstoffe, Stabilisatoren sowie Aromen, Farbpigmente und Farbstoffe zur Herstellung der erfindungsgemässen Arzneimittelformulierungen eingesetzt werden.

Der Anteil an Bindemittel in der Gesamtmischung der Arzneimittelzubereitung ist bevorzugt 0 bis 20% (m/m), der Anteil an Füll- und Hilfsstoffen in der Gesamtmischung beträgt 20 bis 99%, bevorzugt von 50 bis 99%.

Mit dem erfindungsgemäßen Verfahren werden überraschend stabile Adsorbate von Atorvastatin, insbesondere von amorphem Atorvastatin, hergestellt. Diese Atorvastatin-Adsorbate werden als pharmazeutischer Wirkstoff in den erfindungsgemäßen Zubereitungen eingesetzt. Bevorzugt wird Atorvastatin im Rahmen dieser Erfindung in Form seiner Salze eingesetzt. Beispiele der erfindungsgemäß verwendeten Atorvastatin-Salze sind das Mononatrium- und die Monokalium-Salze sowie die Magnesium- und Calcium-Salze sowie deren Hydrate und Solvate. Ganz besonders bevorzugt sind das Atorvastatin-Hemicalcium-Salz und dessen Hydrate und Solvate.

Im Rahmen dieser Erfindung können weiterhin die jeweiligen Hydrate, Solvate, Ester, Lactone und Tautomere des Atorvastatins eingesetzt werden, die insbesondere im Rahmen der Wirkstoffherstellung am Ende des Syntheseweges in Lösung anfallen können, was die Isolation des reinen Wirkstoffes vermeidet.

Es wurde nun erfindungsgemäß ein Verfahren gefunden, das ausgehend von einer Lösung des Atorvastatins oder eines seiner Salze, Hydrate, Solvate, Ester, Lactone und Tautomere in einem organischen Lösungsmittel zu direkt weiterverarbeitbaren Wirkstoff-Absorbaten führt.

Die Atorvastatin-Wirkstofflösung kann grundsätzlich in einer Ausführungsform der Erfindung durch Auflösen des Atorvastatins oder eines seiner Salze, Hydrate, Solvate, Ester, Lactone und Tautomere in einem geeigneten organischen Lösungsmittel hergestellt werden; vorteilhafter ist jedoch, eine im Rahmen der Synthese ohnehin anfallende Wirkstofflösung ohne Isolierung des Atorvastatins direkt zu verwenden.

Beispielsweise kann das Atorvastatin gemäß der EP 409 281 A1, Beispiel 10 hergestellt werden, wobei dann auf den Rekristallisierungsschritt durch Lösen in Ethylacetat und Ausfällen mit Hexan verzichtet wird, und statt dessen das Adsorbermaterial in der Wirkstofflösung suspendiert und das Lösungsmittel durch Trocknen später entfernt wird. Die Art des organischen Lösungsmittels ergibt sich dann im Einzelfall aus dem abschließenden Synthese-Schritt der Wirkstoffherstellung.

Zu dieser organischen Wirkstofflösung wird ein darin nicht bzw. gering löslicher pharmazeutisch akzeptabler Hilfsstoff als Adsorbermaterial zugegeben, gut benetzt und unmittelbar danach das Lösungsmittel durch Trocknen entfernt. Der Trocknungsvorgang kann durch Temperieren und Vakuum unterstützt werden. Er wird vorteilhaft so gestaltet, dass durch geeigneten mechanischen Einfluss (Dreh-, Taumel-, Rührbewegung) eine einheitliche Verteilung gegeben ist. Das Lösungsmittel kann durch Arbeiten im geschlossenen System zurückgewonnen und für den Folgeprozeß wieder eingesetzt werden. Erfindungsgemäß ist, dass eine Ausfällung und Isolierung des Atorvastatins entfällt. Gemäß dem beschriebenen Verfahren hergestellte atorvastatinhaltige Adsorbate können direkt zur Weiterverarbeitung zu Arzneiformen wie Tabletten, Kapseln, Pellets oder Granulaten eingesetzt werden, bevorzugt zur Weiterverarbeitung mittels eines Direktkompressionsverfahrens.

Optional können die so erhaltenen Adsorbate oder Arzneiformen für spezielle Anwendungen weiter mit Überzügen aus pharmazeutischen Polymethacrylaten, wie z. B. Eudragit^{®}-Filme, Methylcellulose, Ethylcellulosen, Hydroxypropylmethylcellulosen, Celluloseacetatphthalaten und/oder Schellack versehen werden, um einen bestimmten Anwendungszweck, z. B. kontrollierte Wirkstoff-Freisetzung, Geschmacksabdeckung zu erzielen. Hierfür stehen dem pharmazeutischen Fachmann ausreichende technische Möglichkeiten zur Verfügung.

Überraschend wurde gefunden, dass nach dem erfindungsgemäßen Verfahren hergestellte Adsorbate den Wirkstoff binden ohne dass es zur Ausbildung von wirkstofftypischen Kristallstrukturen kommt. Dies konnte anhand von röntgendiffraktometrischen Messungen gezeigt werden. Vergleichende Stabilitätsuntersuchungen zeigen zudem, dass die bevorzugten Atorvastatin-Adsorbate eine bessere Stabilität und eine schnellere Auflösegeschwindigkeit aufweisen, als der reine amorphe Wirkstoff. Bestimmte Adsorbermaterialien, z. B. auf Siliziumdioxidbasis weisen stärkere Bindungseigenscharten und damit ein anderes Freisetzungsverhalten des adsorbierten Atorvastatins auf.

Die genannten Eigenschaften bleiben auch insbesondere dann erhalten, wenn die Atorvastatin-Adsorbate zu Arzneiformen, wie z. B. Tabletten verarbeitet werden.

Die Erfindung wird nun anhand der Abbildungen und Beispiele näher erläutert, ohne jedoch die Erfindung darauf zu beschränken.

Es zeigen,
- Abbildung 1: ein Pulverröntgenbeugungsdiagramm eines nicht erfindungsgemäßen Atorvastatin-Mikrokristalline Cellulose-Adsorbates (im Verhältnis 1:1) in der oberen Kurve, sowie als Vergleich in der unteren Kurve mikrokristalline Cellulose alleine,
- Abbildung 2: ein Pulverröntgenbeugungsdiagramm eines erfindungsgemäßen Atorvastatin-Mannitol-Adsorbates (im Verhältnis 1:1) in der oberen Kurve, sowie als Vergleich in der unteren Kurve Mannitol alleine,
- Abbildung 3: ein Pulverröntgenbeugungsdiagramm eines erfindungsgemäßen Atorvastatin-Lactose-Adsorbates (im Verhältnis 1:1) in der oberen Kurve, sowie als Vergleich in der unteren Kurve Lactose alleine,
- Abbildung 4: ein Pulverröntgenbeugungsdiagramm eines nicht erfindungsgemäßen Atorvastatin-Mikrokristalline Cellulose-Adsorbates (im Verhältnis 1:1,5) in der oberen Kurve, sowie als Vergleich in der unteren Kurve mikrokristalline Cellulose alleine,
- Abbildung 5: ein Pulverröntgenbeugungsdiagramm eines erfindungsgemäßen Atorvastatin-Mannitol-Adsorbates (im Verhältnis 1:1) in der oberen Kurve, sowie als Vergleich in der unteren Kurve Mannitol alleine,
- Abbildung 6: ein Pulverröntgenbeugungsdiagramm eines nicht erfindungsgemäßen Atorvastatin-Mikrokristalline Cellulose-Adsorbates (im Verhältnis 1:2), in der oberen Kurve, sowie als Vergleich in der unteren Kurve mikrokristalline Cellulose alleine,

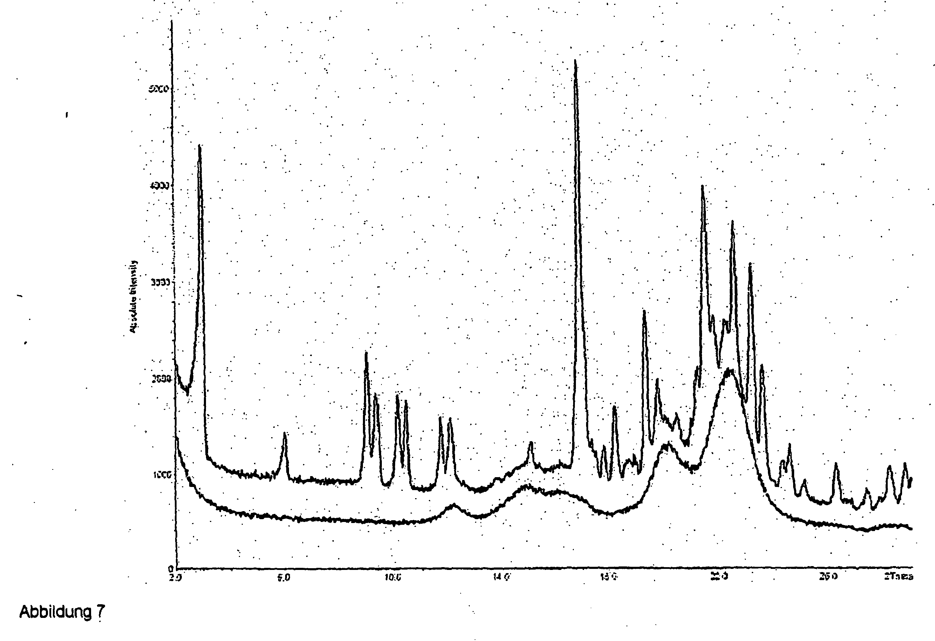

- Abbildung 7: ein Pulverröntgenbeugungsdiagramm von kristallinem Atorvastatin (sogenannte Form I, vergleiche WO 97/03959 A1), im Pulvermischungsverhältnis 1:1 mit mikrokristalliner Cellulose, sowie als Vergleich in der unteren Kurve mikrokristalline Cellulose alleine,

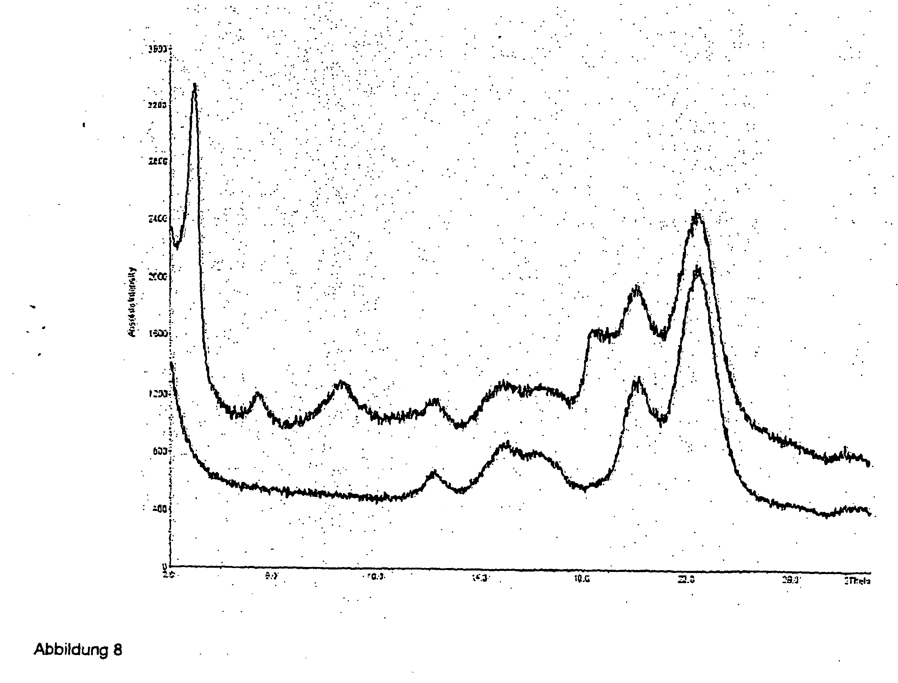

- Abbildung 8: ein Pulverröntgenbeugungsdiagramm von kristallinem Atorvastatin (sogenannte Form V, vergleiche WO 01/36384 A1), im Pulvermischungsverhältnis 1:1 mit mikrokristalliner Cellulose, sowie als Vergleich in der unteren Kurve mikrokristalline Cellulose alleine.

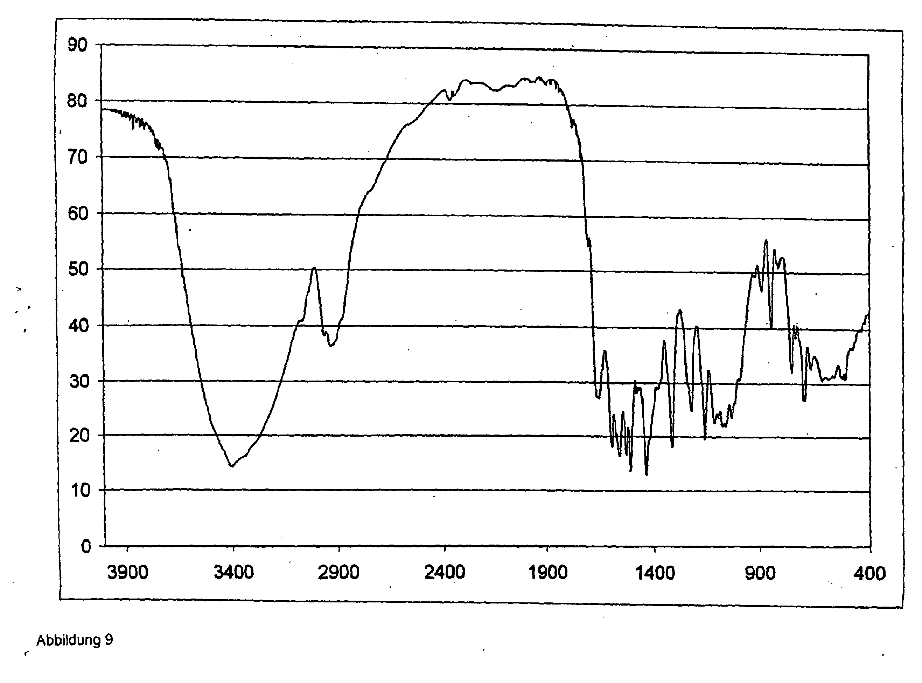

- Abbildung 9: FTIR-Spektrum eines nicht erfindungsgemäßen Atorvastatin-Mikrokristalline Cellulose Adsorbates (im Verhältnis 1:1),

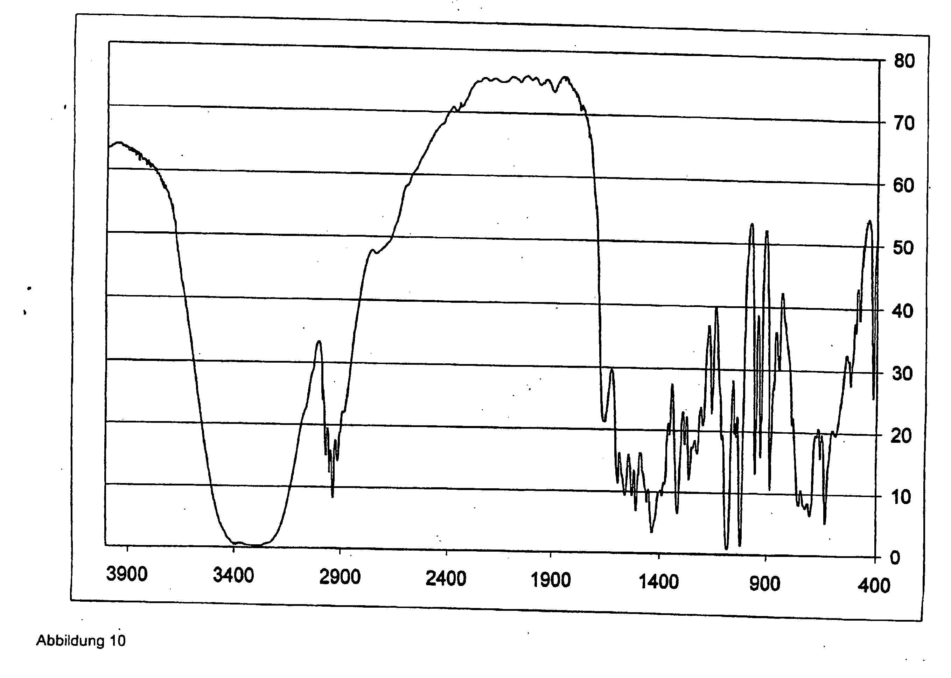

- Abbildung 10: FTIR-Spektrum eines erfindungsgemäßen Atorvastatin-Mannitol-Adsorbates (im Verhältnis 1:1) und

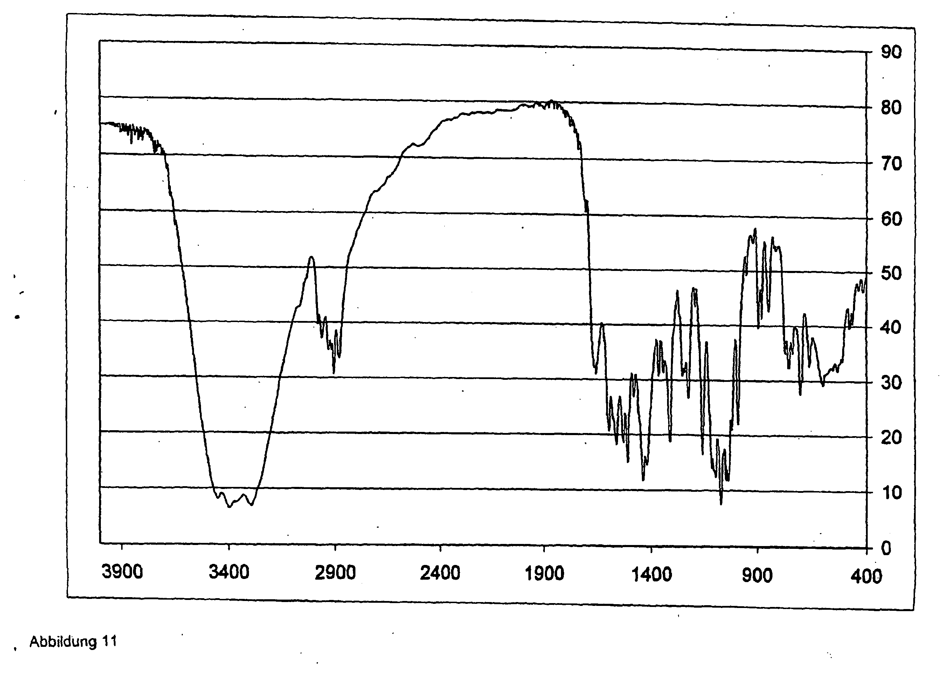

- Abbildung 11: FTIR-Spektrum eines erfindungsgemäßen Atorvastatin-Lactose-Adsorbates (im Verhältnis 1:1).

### Beispiele 1 bis 6

Verwendete apperative Ausstattung der analytischen Untersuchungen:

### HPLC-Messungen:

Alle HPLC-Messungen wurden mit einem Agilent 100-HPLC durchgeführt.

| | |
|---|---|
| Verwendete Säule: | Inertril ODS 2,5µ (150 x 4,6 mm) |
| Mobile Phase: | 55% 0,05M Natriumacetat |
| | 54% Acetonitril |
| | pH: 4,0 (Einstellung mit Essigsäure) |
| Fliessgeschwindigkeit: | 1 ml min⁻¹ |
| Detektor: | UV bei 246 nm |
| Injektionsvolumen: | 20 ml |
| Retentionszeit Atorvastatin: | ca. 15 min |
| Analysedauer: | 60 min |

### Röntgenmessungen:

Alle Pulverröntgenbeugungsdiagramme wurden wie folgt gemessen:

| | |
|---|---|
| Gerät: | STADI P Transmissions-Diffraktometer |
| | Cu-Ka₁-Strahlung (1= 1.54056Å), U = 40 kV, I = 35 mA |
| | Primärstrahl-Monochromator (gekrümmt Ge 111) |
| Detektor: | Linearstellungssensitiv |
| Spaltbreite: | 1 mm |
| Linear PSD: | 2Θ = 2° bis 34°, 25 s / 0,2° stufenweise, Inkrement Δ2Θ = 0,02 |
| Probe: | Pulver in Mylar-Folie |

### IP-Spektren:

| | |
|---|---|
| Gerät: | GENESIS II FTIR-Spektrometer |
| Messmethode: | KBr-Pressling mit 1 % Testsubstanz |

Die Spektren sind als Transmissionswerte (in %) in Abhängigkeit von den Wellenzahlen (cm⁻¹) dargestellt.

### Beispiel 1: Atorvastatin-Mikrokristalline Cellulose-Adsorbat (nicht erfindungsgemäß)

Zu einer Lösung von heterogenem Atorvastatin-Hemicalcium in Aceton (0,15g/ml) werden 0,15g/ml mikrokristalline Cellulose (CelphereSCP-100^{®}) zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

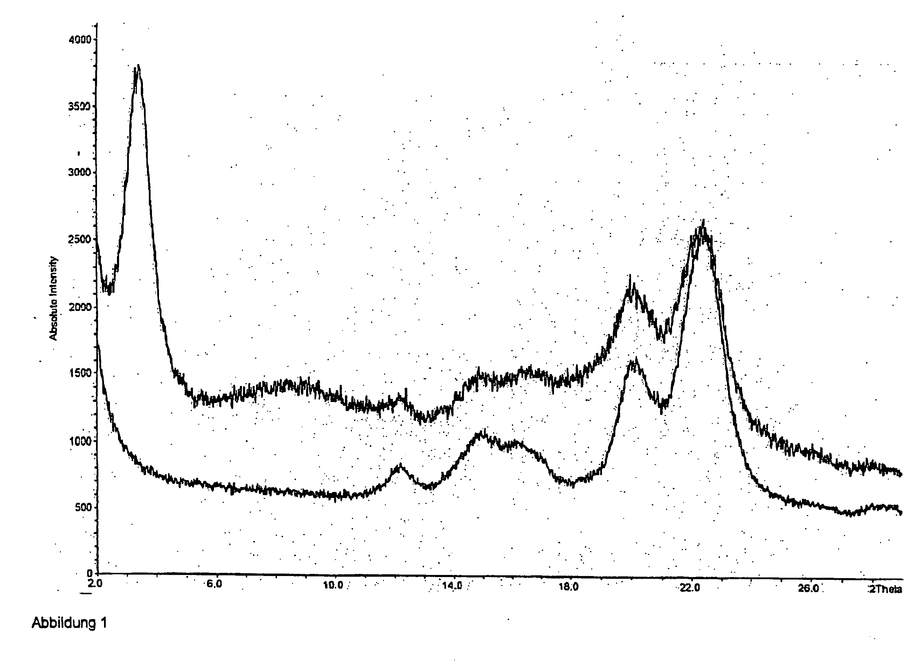

Wirkstoffgehalt des Adsorbates mittels HPLC: 49,6 % (theoretisch 50%)
Pulverröntgenbeugungsdiagramm: Abbildung 1
Verunreinigungsprofil: Summe aller Verunreinigungen: HPLC, in %:

| | Ausgang | 15 Tage (70°C / 75% relative Feuchtigkeit) |
|---|---|---|
| Probe (Adsorbat) | 0,76 | 1,11 |
| Vergleich (amorphes | | |
| Atorvastatin-Calcium) | 1,07 | 1,92 |
| Tablette | 0,77 | 1,07 |

Aus dem Adsorbat wurden Atorvastatin-Tabletten mittels Direktverpressung nach folgender Zusammensetzung hergestellt:
■ Atorvastatin-Mikrokristalline-Cellulose-Adsorbat 80 mg
■ Mikrokristalline Cellulose (CelphereSCP-100^{®}) 408 mg
■ Hilfsstoffe (Croscarmellose-Natrium, Natriumlaurylsulfat, Siliziumdioxid, Magnesiumstearat) in den üblichen Mengen 72 mg

Die verwendeten Mengen der weiteren Hilfsstoffe sind dem Fachmann aufgrund seines Basiswissens bekannt und können Standardwerken zur Formulierung von Tabletten, wie z.B. Ritschel et al., die Tablette, Editio Cantor - Aulendorf, 2. Auflage [2002] entnommen werden.

Eigenschaften der pressfertigen Mischung und der Tablette:
■ Kompressibilität und Fließfähigkeit: gut
■ Mittlere Härte 142 N
■ Abrieb: 0,06% (bestimmt nach Ph. Eur.)
■ Zerfallzeit: 40 sek. (bestimmt nach Ph. Eur.)
■ Freisetzung: 100% nach 5 Min. (Ph. Eur., 1000 ml Wasser, 37°C, Paddel 75 U min⁻¹)

Die so erhaltenen Tabletten können gegebenenfalls mit einem Überzug versehen werden.

### Beispiel 2 : Atorvastatin-Mannitol-Adsorbat (erfindungsgemäß)

Zu einer Lösung von heterogenem Atorvastatin-Hemicalcium in Aceton (0,15 g/ml) werden 0,15g/ml Mannitol *(Mannogern*^{®)} zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

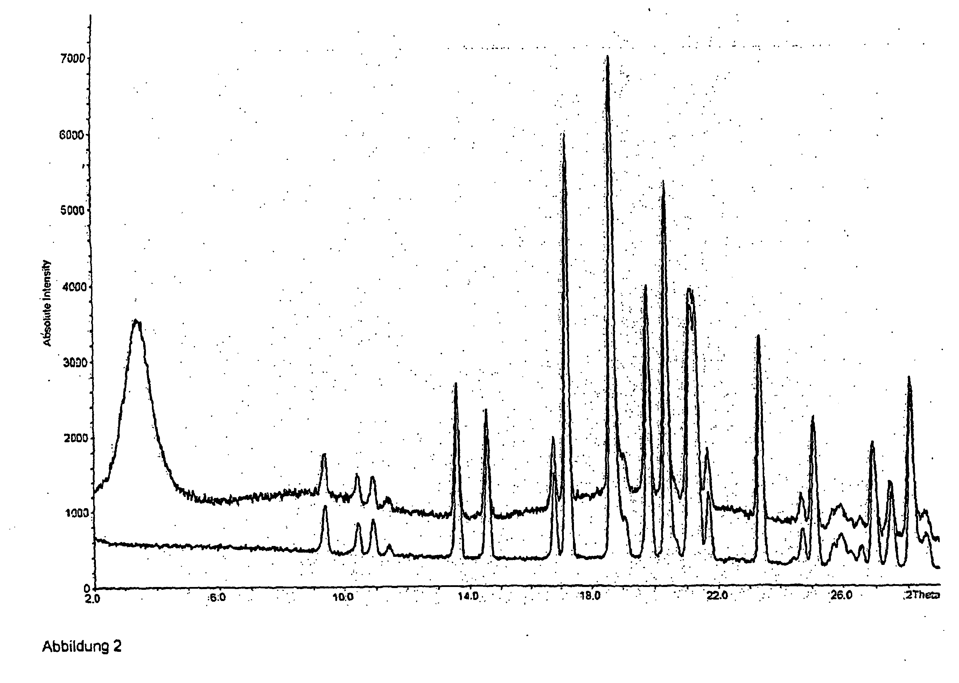

Wirkstoffgehalt des Absorbates, mittels HPLC: 49,85% (theoretisch 50%)
Pulverröntgenbeugungsdiagramm: Abbildung 2
Verunreinigungsprofil (Summe aller Verunreinigungen, HPLC, in %):

| | Ausgang | 15 Tage (70°C / 75% relative Feuchtigkeit) |
|---|---|---|
| Probe (Adsorbat) | 0,91 | 1,40 |
| Vergleich (amorphes | | |
| Atorvastatin-Calcium) | 1,07 | 1,92 |
| Tablette | 0,85 | 1,01 |

Aus dem Adsorbat wurden Atorvastatin-Tabletten mittels Direktverpressung nach folgender Zusammensetzung hergestellt:
■ Atorvastatin-Mannitol-Adsorbat 80 mg
■ Mannitol 408 mg
■ Hilfsstoffe (wie in Bsp. 1) 72 mg

Eigenschaften der pressfertigen Mischung und der Tabletten:
■ Kompressibilität und Fließfähigkeit: befriedigend bis gut
■ Mittlere Härte 153 N
■ Abrieb: 0,18% (bestimmt nach Ph. Eur.)
■ Zerfallzeit: 65 sek. (bestimmt nach Ph. Eur.)
■ Freisetzung: 100% nach 7 Min. (Ph. Eur., 1000 ml Wasser, 37°C, Paddel 75 U min⁻¹)

Die so erhaltenen Tabletten können gegebenenfalls mit einem Überzug versehen werden.

### Beispiel 3: Atorvastatin-Lactose-Adsorbat (erfindungsgemäß)

Zu einer Lösung von heterogenem Atorvastatin-Hemicalcium in Aceton (0,15 g/ml) werden 0,15g/ml Lactose (Lactopress^{®} Anhydrous) zugegeben und gleichmäßig suspendiert. Das Lösungsmittel wird nun unter permanenter Bewegung und Vakuum (Rotationsverdampfer oder Trommeltrockner) bei 25°C abgetrocknet. Die Mischung wird abschließend zur Entfernung von Restlösemittel kurzzeitig bei 35°C nachgetrocknet.

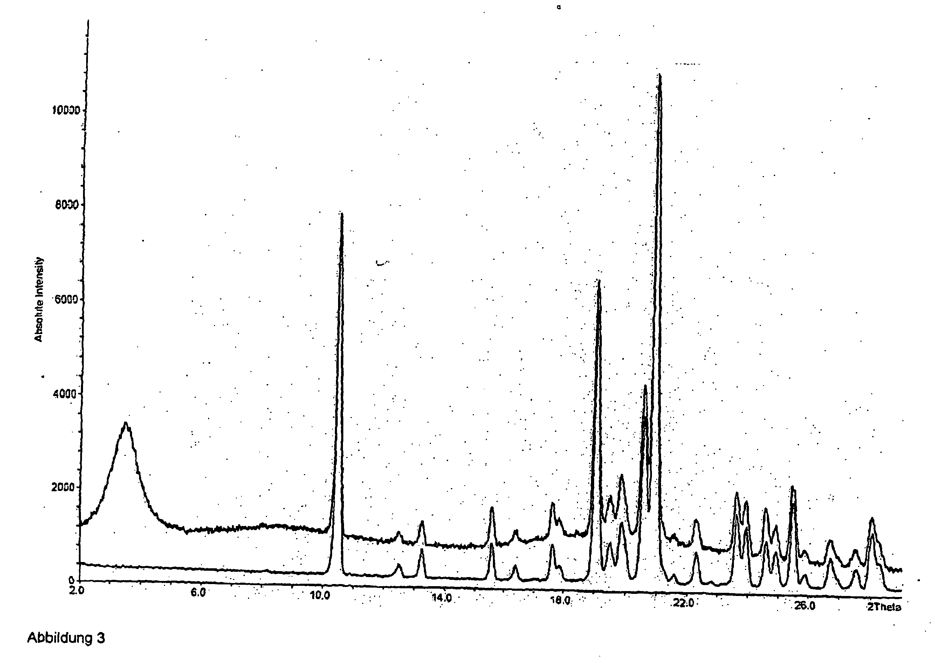

Wirkstoffgehalt des Absorbates mittels HPLC: 51,18% (51,18% (theoretisch 50%)
Pulverröntgenbeugungsdiagramm: Abbildung 3
Verunreinigungsprofil (Summe aller Verunreinigungen, HPLC, in %):

| | Ausgang | 15 Tage (70°C / 75% relative Feuchtigkeit) |
|---|---|---|
| Probe (Adsorbat) | 0,80 | 1,03 |
| Vergleich (amorphes | | |
| Atorvastatin-Calcium) | 1,07 | 1,92 |
| Tablette | 0,79 | 1,05 |

Aus dem Adsorbat wurden Atorvastatin- Tabletten mittels Direktverpressung nach folgender Zusammensetzung hergestellt:
■ Atorvastatin-Lactose-Adsorbat 80 mg
■ Lactose 408 mg
■ Hilfsstoffe (wie in Bsp. 1) 72 mg

Eigenschaften der pressfertigen Mischung und der Tabletten:
■ Kompressibilität und Fließfähigkeit: befriedigend bis gut
■ Mittlere Härte 138 N
■ Abrieb: 0,18% (bestimmt nach Ph. Eur.)
■ Zerfallzeit: 80 sek. (bestimmt nach Ph. Eur.)
■ Freisetzung: 100% nach 8 Min. (Ph. Eur., 1000 ml Wasser, 37°C, Paddel 75 U min⁻¹)

Die so erhaltenen Tabletten können ebenfalls mit einem Überzug versehen werden.

### Beispiel 4: Atorvastatin-Mikrokristalline-Cellulose-Adsorbat (nicht erfindungsgemäß)

Zu einer Lösung von heterogenem Atorvastatin-Hemicalcium in Ethanol (0,15 g/ml) werden 0,225g/ml mikrokristalline Cellulose (Verhältnis Wirkstoff : Adsorbat 2:3) zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

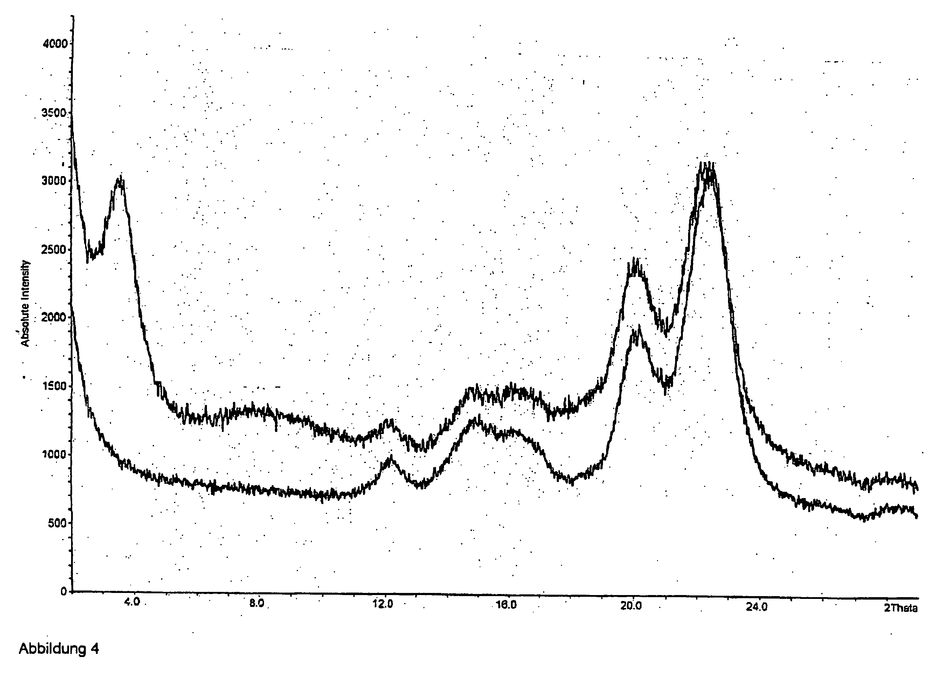

Wirkstoffgehalt des Absorbates mittels HPLC: 40,3%: (theoretisch 40%)
Pulverröntgenbeugungsdiagramm : Abbildung 4
Verunreinigungsprofil (Summe aller Verunreinigungen, HPLC, in %):

| | Ausgang | 15 Tage (70°C / 75% relative Feuchtigkeit) |
|---|---|---|
| Probe (Adsorbat) | 0,83 | 1,04 |
| Vergleich (amorphes | | |
| Atorvastatin-Calcium) | 1,07 | 1,92 |

### Beispiel 5: Atorvastatin-Mannitol-Adsorbat (erfindungsgemäß)

Zu einer Lösung von heterogenem Atorvastatin-Hemicalcium in Ethanol (0,15 g/ml) werden 0,15 g/ml Mannitol (1:1 Mischung) zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

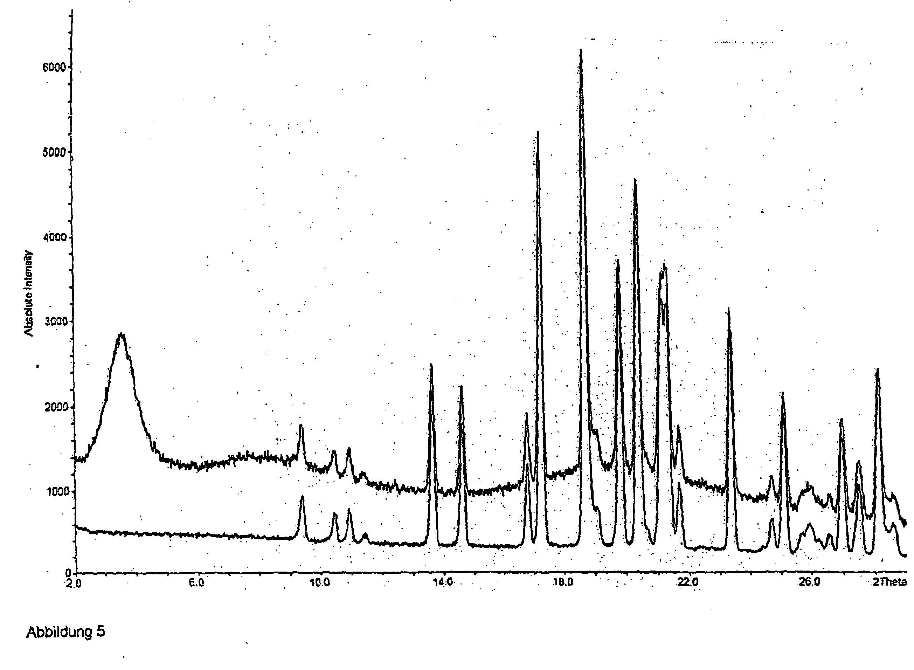

Wirkstoffgehalt des Absorbates mittels HPLC: 50,4% (theoretisch 50%)
Pulverröntgenbeugungsdiagramm: Abbildung 5
Verunreinigungsprofil (Summe aller Verunreinigungen, HPLC, in %):

| | Ausgang | 15 Tage (70°C / 75% relative Feuchtigkeit) |
|---|---|---|
| Probe (Adsorbat) | 0,72 | 1,05 |
| Vergleich (amorphes | | |
| Atorvastatin-Calcium) | 1,07 | 1,92 |

### Beispiel 6: Atorvastatin-Mikrokristalline Cellulose-Adsorbat (nicht erfindungsgemäß)

Zu einer Lösung von heterogenem Atorvastatin-Hemicalcium in Ethanol (0,15 g/ml) werden 0,30g/ml mikrokristalline Cellulose (Verhältnis Wirkstoff : Adsorbat 1:2) zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

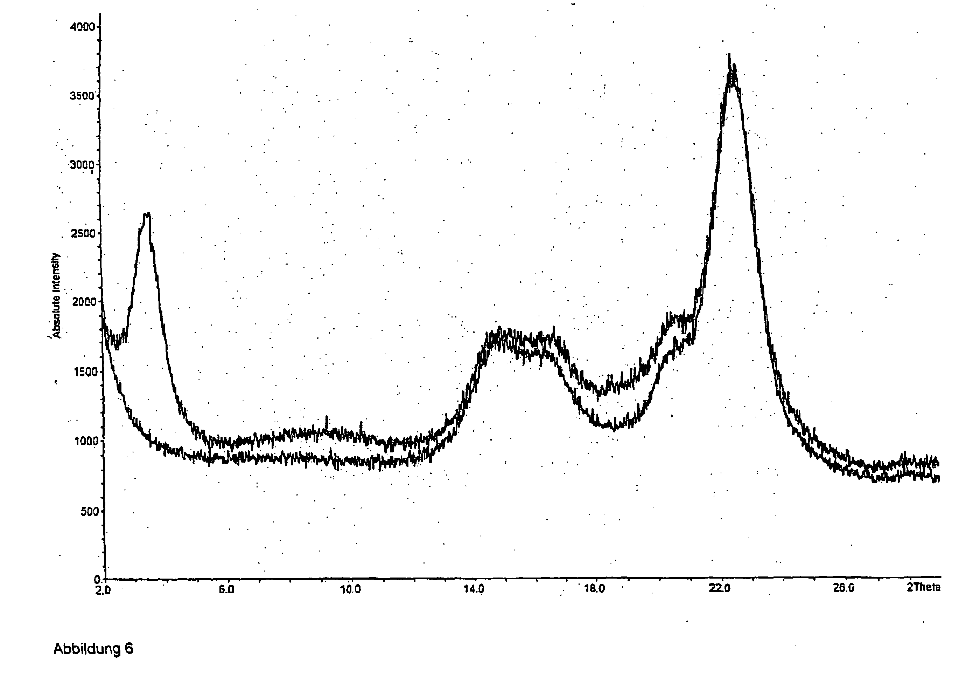

Wirkstoffgehalt des Absorbates mittels HPLC: 33,4% (theoretisch 33,3%)
Pulverröntgenbeugungsdiagramm: Abbildung 6
Verunreinigungsprofil (Summe aller Verunreinigungen, HPLC, in %):

| | Ausgang | 15 Tage (70°C / 75% relative Feuchtigkeit) |
|---|---|---|
| Probe (Adsorbat) | 0,91 | 1,09 |
| Vergleich (amorphes | | |
| Atorvastatin-Calcium) | 1,07 | 1,92 |

## Patentansprüche

1. Verfahren zur Herstellung von Atorvastatin-Adsorbaten und von dessen Solvaten, **dadurch gekennzeichnet, dass** man von einer Lösung des Atorvastatins oder einem seiner Salze, Hydrate, Solvate, Ester, Lactone und Tautomere in wenigstens einem organischen Lösungsmittel mit einem Gesamtwassergehalt von nicht mehr als 10 Vol.-%, bevorzugt von nicht mehr als 5 Vol.-%, ausgeht, Adsorbermaterial, ausgewählt aus der Gruppe, bestehend aus Polyolen und Zuckern oder Mischungen daraus, darin suspendiert und das Lösungsmittel durch Trocknen entfernt.

2. Verfahren gemäß Anspruch 1, **dadurch gekennzeichnet, dass** das Adsorbermaterial aus der Gruppe aus Lactose, Mannitol und Sorbit ausgewählt ist.

3. Verfahren gemäß Anspruch 1 oder 2, **dadurch gekennzeichnet, dass** die Atorvastatin-Adsorbate den Wirkstoff in fein verteilter, amorpher Form und gegebenenfalls als dessen Solvate enthalten.

4. Verfahren gemäß Anspruch 1 bis 3, **dadurch gekennzeichnet, dass** man ein Verhältnis von Wirkstoff zu Adsorbat im Bereich von 1 : 0,1 bis 10, insbesondere im Bereich von 1:1 bis 1:2 einstellt.

5. verfahren gemäß einem oder mehreren der vorhergehenden Ansprüche 1 bis 4, **dadurch gekennzeichnet, dass** man als Lösungsmittel organische Lösungsmittel mit einem Gesamtwasseranteil von nicht mehr als 10-Vol.%, alleine oder in Mischungen einsetzt, wobei die organischen Lösungsmittel ausgewählt sind aus der Gruppe der niederen Alkanole mit 1 bis 4 Kohlenstoffatomen, der Gruppe der Ether und der Gruppe der aliphatischen Ketone und deren Gemischen.

6. Verfahren gemäß Anspruch 5, **dadurch gekennzeichnet, dass** man als Lösungsmittel Aceton, Ethanol, Methanol, Methylethylketon oder deren Gemische einsetzt.

7. Verfahren gemäß einem der Ansprüche 1 bis 6, **dadurch gekennzeichnet, dass** man eine Wirkstofflösung einsetzt, die im Rahmen der Atorvastatinsynthese anfällt und darin das Adsorbermaterial suspendiert.

8. Atorvastatin-Adsorbate und dessen Solvate, hergestellt durch ein Verfahren mit den Schritten:
- Bereitstellen einer Lösung des Atorvastatins oder einem seiner Salze, Hydrate, Solvate, Ester, Lactone und Tautomere in wenigstens einem organischen Lösungsmittel mit einem Gesamtwassergehalt von nicht mehr als 10 Vol.-%, bevorzugt von nicht mehr als 5 Vol.-%;
- Suspendieren eines Adsorbermaterials in der Lösung, wobei das Adsorbermaterial ausgewählt ist aus der Gruppe, bestehend aus Polyolen und Zuckern oder Mischungen daraus; und
- Entfernen des Lösungsmittels durch Trocknen.

9. Atorvastatin-Adsorbate und dessen Solvate gemäß Anspruch 8, **dadurch gekennzeichnet, dass** das Adsorbermaterial aus der Gruppe aus Lactose, Mannitol und Sorbit ausgewählt ist.

10. Atorvastatin-Adsorbate und dessen Solvate gemäß Anspruch 8 oder 9, **dadurch gekennzeichnet, dass** die Atorvastatin-Adsorbate den Wirkstoff in fein verteilter, amorpher Form und gegebenenfalls als dessen Solvate enthalten.

11. Atorvastatin-Adsorbate und dessen Solvate gemäß einem oder mehreren der vorhergehenden Ansprüche 8 bis 10, **dadurch gekennzeichnet, dass** ein Verhältnis von Wirkstoff zu Adsorbat im Bereich von 1 : 0,1 bis 10, insbesondere im Bereich von 1:1 1 bis 1:2 einstellt ist.

12. Atorvastatin-Adsorbate und dessen Solvate gemäß einem oder mehreren der vorhergehenden Ansprüche 8 bis 11, **dadurch gekennzeichnet, dass** als Lösungsmittel organische Lösungsmittel mit einem Gesamtwasseranteil von nicht mehr als 10-Vol.%, alleine oder in Mischungen einsetzt worden ist, wobei die organischen Lösungsmittel ausgewählt sind aus der Gruppe der niederen Alkanole mit 1 bis 4 Kohlenstoffatomen, der Gruppe der Ether und der Gruppe der aliphatischen Ketone und deren Gemischen.

13. Atorvastatin-Adsorbate und dessen Solvate gemäß Anspruch 12, **dadurch gekennzeichnet, dass** als Lösungsmittel Aceton, Ethanol, Methanol, Methylethylketon oder deren Gemische einsetzt wurde.

14. Atorvastatin-Adsorbate und dessen Solvate gemäß einem der Ansprüche 8 bis 13, **dadurch gekennzeichnet, dass** das Atorvastatin-Adsorbat den Wirkstoff in fein verteilter amorpher Form, bevorzugt als Alkalimetall- oder als Erdalkalimetallsalz sowie deren Hydrate und Solvate und besonders bevorzugt als Calciumsalz sowie dessen Hydrate enthält.

15. Pharmazeutische Formulierung mit zumindest einem Wirkstoff und gegebenenfalls weiteren pharmazeutisch akzeptablen Hilfsstoffen, **dadurch gekennzeichnet, dass** sie als Wirkstoff die Atorvastatin-Adsorbate gemäß einem der Ansprüche 8 bis 14 enthält.

16. Pharmazeutische Formulierung gemäß Anspruch 15, **dadurch gekennzeichnet, dass** sie in Form von Tabletten, Kapseln, Pellets und Granulaten vorliegt, die auf bekannte Weise mit üblichen pharmazeutisch akzeptablen Hilfsstoffen herstellbar ist.

17. Pharmazeutische Formulierung gemäß Anspruch 15 oder 16 in Form von den Wirkstoff schnell freisetzenden, durch Direktverpressung hergestellten Tabletten.

## Claims

1. Method for the preparation of atorvastatin adsorbates and solvates thereof, **characterized in that** one starts from a solution of the atorvastatin or one of its salts, hydrates, solvates, esters, lactones and tautomers in at least one organic solvent having a total water content of not more than 10 vol.-%, preferably not more than 5 vol.-%, suspending therein an adsorber material selected from the group consisting of polyols and sugars or mixtures thereof, and removing the solvent by drying.

2. Method according to claim 1, **characterized in that** the adsorber material is selected from the group of lactose, mannitol and sorbitol.

3. Method according to claim 1 or 2, **characterized in that** the atorvastatin adsorbates comprise the active ingredient in finely dispersed amorphous form, and if required, as the solvates thereof.

4. Method according to claim 1 to 3, **characterized in that** a ratio of active ingredient to adsorbate is adjusted in the range of from 1 : 0.1 to 10, especially in the range of from 1:1 to 1:2.

5. Method according to one or more of the preceding claims 1 to 4, **characterized in that** organic solvents having a total water amount of not more than 10 vol.-%, alone or in mixtures, are used as solvent, wherein the organic solvents are selected from the group of the lower alkanols having 1 to 4 carbon atoms, the group of the ethers and the group of the aliphatic ketones and mixtures thereof.

6. Method according to claim 5, **characterized in that** acetone, ethanol, methanol, methyl ethyl ketone or mixtures thereof is/are used as solvent.

7. Method according to one of the claims 1 to 6, **characterized in that** an active ingredient solution which arises within the scope of the atorvastatin synthesis is used, and suspending the adsorber material therein.

8. Atorvastatin adsorbates and solvates thereof, prepared by a method having the steps of:
- providing a solution of the atorvastatin or one of its salts, hydrates, solvates, esters, lactones and tautomers in at least one organic solvent having a total water content of not more than 10 vol.-%, preferably not more than 5 vol.-%;
- suspending an adsorber material in the solution, wherein the adsorber material is selected from the group consisting of polyols and sugars or mixtures thereof; and
- removing the solvent by drying.

9. Atorvastatin adsorbates and solvates thereof according to claim 8, **characterized in that** the adsorber material is selected from the group of lactose, mannitol and sorbitol.

10. Atorvastatin adsorbates and solvates thereof according to claim 8 or 9, **characterized in that** the atorvastatin adsorbates comprise the active ingredient in finely dispersed amorphous form, and if required, as the solvates thereof.

11. Atorvastatin adsorbates and solvates thereof according to one or more of the preceding claims 8 to 10, **characterized in that** a ratio of active ingredient to adsorbate is adjusted in the range of from 1 : 0.1 to 10, especially in the range of from 1:1 to 1:2.

12. Atorvastatin adsorbates and solvates thereof according to one or more of the preceding claims 8 to 11, **characterized in that** organic solvents having a total water amount of not more than 10 vol.-%, alone or in mixtures, have been used as solvent, wherein the organic solvents are selected from the group of the lower alkanols having 1 to 4 carbon atoms, the group of the ethers and the group of the aliphatic ketones and mixtures thereof.

13. Atorvastatin adsorbates and solvates thereof according to claim 12, **characterized in that** acetone, ethanol, methanol, methyl ethyl ketone or mixtures thereof has been used as solvent.

14. Atorvastatin adsorbates and solvates thereof according to one of claims 8 to 13, **characterized in that** the atorvastatin adsorbate comprises the active ingredient in finely dispersed amorphous form, preferably as alkali metal salt or as alkaline earth metal salt as well as the hydrates and solvates thereof and especially preferred as calcium salt as well as the hydrates thereof.

15. Pharmaceutical formulation having at least one active ingredient, and if required, further pharmaceutically acceptable adjuvants, **characterized in that** it comprises the atorvastatin adsorbates according to one of claims 8 to 14 as active ingredient.

16. Pharmaceutical formulation according to claim 15, **characterized in that** it is in form of tablets, capsules, pellets and granulates which are preparable in a known manner with usual pharmaceutically acceptable adjuvants.

17. Pharmaceutical formulation according to claim 15 or 16 in form of tablets which rapidly release the active ingredient and are prepared by direct pressing.

## Revendications

1. Procédé de fabrication d'adsorbats d'atorvastatine et de leurs solvates, **caractérisé en ce que** l'on part d'une solution d'atorvastatine ou d'un de ses sels, hydrates, solvates, esters, lactones et tautomères dans au moins un solvant organique ayant une teneur en eau totale de pas plus de 10 % en volume, de préférence de pas plus de 5 % en volume, que l'on y met en suspension un matériau adsorbant choisi dans le groupe constitué des polyols et des sucres ou mélanges de ceux-ci, et que l'on élimine le solvant par séchage.

2. Procédé selon la revendication 1, **caractérisé en ce que** le matériau adsorbant est choisi dans le groupe constitué de lactose, mannitol et sorbitol.

3. Procédé selon la revendication 1 ou 2, **caractérisé en ce que** les adsorbats d'atorvastatine contiennent le principe actif sous une forme amorphe finement répartie, et le cas échéant en tant que leurs solvates.

4. Procédé selon la revendication 1 à 3, **caractérisé en ce que** l'on règle le rapport entre le principe actif et l'adsorbat dans la plage de 1/0,1 à 10, en particulier dans la plage de 1/1 à 1/2.

5. Procédé selon une ou plusieurs des revendications précédentes 1 à 4, **caractérisé en ce que** l'on utilise comme solvant des solvants organiques ayant une proportion en eau totale de pas plus de 10 % en volume, seuls ou en mélanges, les solvants organiques étant choisis dans le groupe des alcanols inférieurs comportant de 1 à 4 atomes de carbone, le groupe des éthers et le groupe des cétones aliphatiques et leurs mélanges.

6. Procédé selon la revendication 5, **caractérisé en ce que** l'on met en oeuvre comme solvant de l'acétone, de l'éthanol, du méthanol, de la méthyléthylcétone ou leurs mélanges.

7. Procédé selon l'une des revendications 1 à 6, **caractérisé en ce que** l'on met en oeuvre une solution de principe actifs qui apparaît dans le cadre de la synthèse de l'atorvastatine et que l'on y met le matériau adsorbant en suspension.

8. Adsorbats d'atorvastatine et leurs solvates, fabriqués d'après un procédé composé des étapes suivantes :
- préparation d'une solution d'atorvastatine ou d'un de ses sels, hydrates, solvates, esters, lactones et tautomères dans au moins un solvant organique ayant une teneur en eau totale de pas plus de 10 % en volume, de préférence de pas plus de 5 % en volume ;
- suspension dans la solution d'un matériau adsorbant choisi dans le groupe constitué des polyols et des sucres ou mélanges de ceux-ci ; et
- élimination du solvant par séchage.

9. Adsorbats d'atorvastatine et leurs solvates selon la revendication 8, **caractérisés en ce que** le matériau adsorbant est choisi dans le groupe constitué de lactose, mannitol et sorbitol.

10. Adsorbats d'atorvastatine et leurs solvates selon la revendication 8 ou 9, **caractérisés en ce que** les adsorbats d'atorvastatine contiennent le principe actif sous une forme amorphe finement répartie, et le cas échéant en tant que leurs solvates.

11. Adsorbats d'atorvastatine et leurs solvates selon une ou plusieurs des revendications précédentes 8 à 10, **caractérisés en ce que** l'on règle le rapport entre le principe actif et l'adsorbat dans la plage de 1/0,1 à 10, en particulier dans la plage de 1/1 à 1/2.

12. Adsorbats d'atorvastatine et leurs solvates selon une ou plusieurs des revendications précédentes 8 à 11, **caractérisés en ce que** l'on a utilisé comme solvant des solvants organiques ayant une proportion en eau totale de pas plus de 10 % en volume, seuls ou en mélanges, les solvants organiques étant choisis dans le groupe des alcanols inférieurs comportant de 1 à 4 atomes de carbone, le groupe des éthers et le groupe des cétones aliphatiques et leurs mélanges.

13. Adsorbats d'atorvastatine et leurs solvates selon la revendication 12, **caractérisés en ce que** l'on a mis en oeuvre comme solvant de l'acétone, de l'éthanol, du méthanol, de la méthyléthylcétone ou leurs mélanges.

14. Adsorbats d'atorvastatine et leurs solvates selon l'une des revendications 8 à 13, **caractérisés en ce que** l'adsorbat d'atorvastatine contient le principe actif sous une forme amorphe et finement répartie, de préférence en tant que sel de métal alcalin ou sel de métal alcalino-terreux et leurs hydrates et solvates, et notamment de préférence en tant que sel de calcium et ses hydrates.

15. Formulation pharmaceutique avec au moins un principe actif et le cas échéant d'autres auxiliaires pharmaceutiquement acceptables, **caractérisée en ce que** l'on met en oeuvre à titre de principe actif les adsorbats d'atorvastatine selon l'une des revendications 8 à 14.

16. Formulation pharmaceutique selon la revendication 15, **caractérisée en ce qu'**elle se présente sous forme de comprimés, capsules, granules et granulés, et qu'elle peut être fabriquée de manière connue avec des auxiliaires usuels pharmaceutiquement acceptables.

17. Formulation pharmaceutique selon la revendication 15 ou 16, sous forme de comprimés libérant rapidement le principe actif et fabriqués par compression directe.
